# EUROPEAN PATENT SPECIFICATION

(11) **EP 1 483 428 B1**
(45) Date of publication and mention of the grant of the patent: **17.10.2007**
(21) Application number: 03737390.9
(22) Date of filing: 13.01.2003
(51) Int. Cl.: C23C 16/27, C23C 16/56, G01N 33/543, G01N 27/30

(54) **DIAMOND ELECTRODE**
DIAMANTELEKTRODE
ELECTRODE DE DIAMANT

(30) Priority: 05.02.2002 ZA 200200999
(43) Date of publication of application: 08.12.2004
(73) Proprietor: ELEMENT SIX (PTY) LTD, 1559 Springs (ZA)
(72) Inventor: DAVIES, Geoffrey, John, 2194 Randburg (ZA); CHAPMAN, Raymond, Albert, 2001 Johannesburg (ZA); RAS, Anine, Hester, 1609 Edenvale (ZA); NAIDOO, Kaveshini, 1609 Elma Park (ZA)
(74) Representative: Senior, Alan Murray
(86) International application number: PCT/IB2003/000049
(87) International publication number: WO 2003/066930

(56) References cited:
- WO-A-01/25508
- US-A- 5 399 247
- US-A- 5 777 372
- YOSHIMURA M ET AL: "Electrochemical characterization of nanoporous honeycomb diamond electrodes in non-aqueous electrolytes" 11TH EUROPEAN CONFERENCE ON DIAMOND, DIAMOND-LIKE MATERIALS, CARBON NANOTUBES, NITRIDES AND SILICON CARBIDE (DIAMOND 2000), PORTO, PORTUGAL, 3-8 SEPT. 2000, vol. 10, no. 3-7, pages 620-626, XP002239837 Diamond and Related Materials, March-July 2001, Elsevier, Switzerland ISSN: 0925-9635
- HONDA K ET AL: "Impedance characteristics of the nanoporous honeycomb diamond electrodes for electrical double-layer capacitor applications" JOURNAL OF THE ELECTROCHEMICAL SOCIETY, JULY 2001, ELECTROCHEM. SOC, USA, vol. 148, no. 7, pages A668-A679, XP002239838 ISSN: 0013-4651

## Description

### BACKGROUND OF THE INVENTION

THIS invention relates to a method of making a diamond electrode.

The use of electrically conducting diamond as an electrode material is well established. Such diamond electrodes are very versatile and have a wide range of electrochemical applications which include the selective detection and measurement of both inorganic (e.g. heavy metals and cyanides) and organic compounds (e.g. biosensor applications), wastewater treatment (e.g. reduction of nitrates), fuel cell electrodes, and the generation of ozone. The wide applicability of the diamond electrode is due to its unique properties: mechanical strength, chemical inertness, low background interference (high signal to noise ratio) and wide potential window.

Generally, diamond electrodes are made by the chemical vapour deposition (CVD) of diamond or diamond-like carbon onto a suitable substrate, such as a plate or wire, with the diamond being doped by a suitable element, such as boron, to render it electrically conducting. The deposition of boron-doped diamond layers on a substrate by a chemical vapour deposition method (CVD) is taught by, for example, European Patent number 0 518 532 and US Patent number 5,635,258. The synthesis of boron-containing diamonds by a high pressure, high temperature (HPHT) solvent/catalyst method is taught by US Patent Number 4,042,673.

The use of diamond electrodes in analytical and other chemical applications is covered extensively in both the patent and open literature. For example, US Patent number 5,399,247 describes the use of a diamond electrode for the treatment of waste water, PCT Application WO 01/98766 teaches the use of a diamond electrode in the quantitative analysis of xanthin type compounds, PCT Application WO 01/25508 discloses the production of peroxopyrosulphuric acid with a diamond electrode, and US Patent number 6,106,692 teaches a method of quantitative analysis of a plurality of target substances using a diamond electrode.

The effect of boron content on the electrochemical properties of diamond electrodes is also well known (see for example New Diamond and Frontier Carbon Technology vol 9, number 3, pp 189-206 1999).

A disadvantage of CVD diamond electrodes is that the CVD process is expensive and the resulting electrodes are therefore also expensive. There is a need to be able to provide diamond electrodes which are cheaper than CVD diamond electrodes without significantly sacrificing the desirable properties of the electrodes such as robustness and sensitivity.

Yoshimura M et al: "Electrochemical characterization of nanoporous honeycomb diamond electrodes in non-aqueous electrolytes" 11TH EUROPEAN CONFERENCE ON DIAMOND, DIAMOND-LIKE MATERIALS, CARBON NANOTUBES, NITRIDES AND SILICON CARBIDE (DIAMOND 2000), PORTO, PORTUGAL, 3-8 SEPT. 2000, vol. 10, no. 3-7, pages 620-626, Diamond and Related Materials, March-July 2001, Elsevier, Switzerland and Honda K et al: "Impedance characteristics of the nanoporous honeycomb diamond electrodes for electrical double-layer capacitor applications" JOURNAL OF THE ELECTROCHEMICAL SOCIETY, JULY 2001, ELECTROCHEM. SOC, USA, vol. 148 no. 7, PAGES A668-A679 both describe the characteristics of nanoporous honeycomb diamond electrodes. In both references boron-doped polycrystalline diamond thin films were deposited on silicon substrates using microwave assisted plasma chemical vapour deposition techniques.

Both documents are silent as to the characteristics of porous diamond electrodes comprising a polycrystalline mass of diamond particles bonded together and the advantages which accrue from such a product derived by such process.

### SUMMARY OF THE INVENTION

According to a first aspect of the invention, there is provided a diamond electrode comprising a polycrystalline mass of diamond particles bonded together and having a contact surface that is, at least partly, porous.

The porous surface of the electrode is created by leaching non- diamond material, such as a second phase of a metallic material, at least in part, from a bonded polycrystalline mass of diamond particles, either before or after shaping it into an electrode.

Alternatively, or additionally, the porous surface of the electrode is created by subjecting a mass of diamond particles to conditions of elevated temperature and pressure to self-bond the particles together in the absence of a second phase.

Typically, the entire diamond electrode is porous.

The diamond particles may contain an appropriate level of an element other than carbon to render the diamond particles electrically conducting such as, for example, boron.

The diamond particles may be made by a high pressure, high temperature (HPHT) method, by a chemical vapour deposition (CVD) method or may be natural diamond, or may be made by any other method producing diamond particles which are electrically conducting.

The diamond particles may be provided by crushing larger diamond particles or crystals to an appropriate size range. The size of the diamond particles will generally be less than about 1000 microns, preferably less than about 100 microns, and more preferably less than about 60 microns.

The invention extends to the use of a porous diamond electrode of the invention in a biosensor, in particular for use in a bio-recognition system, such as that for enzymes or antibodies, for example.

### BRIEF DESCRIPTION OF THE DRAWINGS

- Figure 1: is a pressure/temperature graph illustrating an example of a temperature-pressure profile useful in making porous diamond electrodes of this invention; and
- Figure 2: is a cyclic voltammogram comparing the responses of a standard diamond electrode with a porous diamond electrode in a perchloric acid system.

### DESCRIPTION OF EMBODIMENTS

The diamond electrode of the invention is characterized by being a polycrystalline mass of diamond particles bonded together and shaped into an electrode having a porous surface, or at least partly porous surface. Typically, the entire diamond electrode is porous.

The porous diamond electrode of the invention is manufactured by causing a mass of diamond particles to be bonded together to form a compact, leaching from the compact any non-diamond material, at least in part, and shaping the compact into the form of an electrode. The step of leaching from the compact any non-diamond material and the step of shaping the compact into the form of an electrode may be interchanged.

Alternatively, or additionally, the porous diamond electrode is manufactured by subjecting a mass of diamond particles to conditions of elevated temperature and pressure to self-bond the particles together in the absence of a second phase, and shaping the self-bonded compact so produced into an electrode. There may be substantial plastic deformation of the diamond particles in the bonded mass.

The diamond particles may be natural diamond, high pressure high temperature (HPHT) synthetic diamond, or chemical vapour deposited (CVD) diamond or any combination of these. The particle size range of the diamond is selected according to the desired internal surface area, average pore size and porosity of the compact. Generally, the maximum particle size of the diamond particles will be 1000 microns, preferably less than 100 microns, and more preferably less than 60 microns. Generally, a larger particle size results in a smaller internal surface area, a larger average pore size and a higher porosity, and a smaller particle size results in a higher internal surface area, a smaller average pore size and a smaller porosity. Diamond particles of the appropriate size may be provided by crushing and grading diamond particles of a larger size.

The diamond particles may be electrically conducting.

The mass of diamond particles may be mixed with a diluent. A diluent is any material which is stable under the conditions at which the mass of diamonds is treated and which may be leached, at least in part, from the diamond compact. A diluent allows the internal surface area, the average pore size and the porosity to be varied independently of the particle size. The nature and physical properties of the diluent, such as particle size, are selected to attain the desired internal surface area, average pore size and porosity. Examples of such diluents are calcium carbonate and magnesium oxide. Preferably, the diluent does not, under the conditions of treatment, either react with the diamond particles or act as a solvent/catalyst. Generally, the diluent when used may be present to a maximum of about 40% by volume of the electrode before any leaching process.

The mass of diamonds may be placed on a substrate. In the case of said substrate being used, the substrate may be removed from the diamond compact after treatment. Alternatively, the substrate may be allowed to remain in place to provide an electrical contact to the diamond compact.

The substrate may be in the form of a plate or disc.

In another embodiment, the substrate may be substantially enclosed within the diamond compact. In this case, the substrate may take the form of a disc, plate, grid, wire or the like. The substrate provides an electrical contact to the diamond compact, and that part of the substrate not enclosed within the diamond compact provides means by which contact to an external circuit may be made.

The porosity and pore size distribution of the compact are controlled, but not exclusively, by the selection of size distribution of the diamond particles, the size distribution and proportion of any diluent, as well as the final conditions of temperature and pressure and the time for which these are maintained.

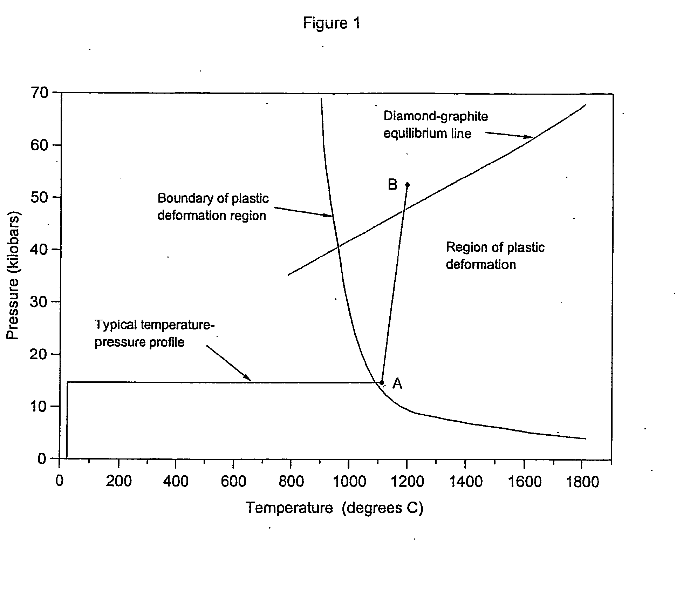

The self-bonding of the diamond particles and the extent of self-bonding is achieved by the selection of the temperature-pressure path and the final conditions of temperature and pressure, as well as the time for which these final conditions are maintained. It is believed that self-bonding of the particles occurs due to the very high contact pressure generated when an asperity on one particle bears upon a substantially flat area of an adjacent particle. The very high contact pressure is well in excess of the nominal applied pressure of the pressurising system. Such high contact pressure when applied at an elevated temperature causes plastic deformation at the contact points between particles thereby promoting the movement of the constituent atoms of the crystal and facilitating self-bonding. Generally, the extent of self-bonding is determined by the selected final conditions of temperature and pressure and the period for which the conditions are applied as is well known in the art of sintering. The temperature-pressure profile to reach the desired final conditions of temperature and pressure (Figure 1) is such that the diamond particles are raised to a condition in the region of plastic deformation for diamond (e.g. point A) as rapidly as possible, before further raising the conditions to those at which the self-bonding takes place (e.g. point B). Such a profile maximises the time spent in the region of plastic deformation and hence maximises the plastic deformation of the particles prior to self-bonding.

The boundary of the plastic deformation region shown in Figure 1 exemplifies but does not define the boundary between the region of plastic deformation and the region of no plastic deformation. It is a transition region and not a sharp cut-off. Furthermore, the displacement of the boundary to higher or lower temperatures depends upon the nature of the impurities in the diamond particle (e.g. boron or nitrogen) and the level of those impurities.

The conditions under which the mass of diamonds is treated will generally be in the region of thermodynamic stability of diamond in the carbon phase diagram. Conditions outside the region of diamond thermodynamic stability may also be used provided that the time for which the conditions are applied is insufficient for significant reversion of diamond to graphite to take place.

The compact may be shaped by any convenient method, such as electrical discharge machining (EDM) and laser cutting.

Non-diamond material may be leached from the compact using suitable methods and reagents. These include dissolution at elevated temperature and pressure.

In a further embodiment, the pores of the compact may be filled, partially or completely, by the infiltration of a suitable insulating material, such as polytetrafluoroethylene. In the case of the pores being filled by an insulating material, surplus material is removed from the surfaces of the compact to expose the diamond. Said infiltration may be performed after either the leaching step or the compact shaping step.

The electrical contact may be provided by a surface layer bonded to the diamond during the heat treatment step or at any convenient step thereafter. The electrical contact may be provided by a wire embedded in the diamond compact during the heat treatment step or at any convenient step thereafter.

The diamond compact may be provided with an electrical contact simultaneously with the bonding step or at a step thereafter.

A plurality of electrodes may be assembled to form a panel. Such panel may be planar or non-planar. The electrodes forming said panel may be connected electrically in series or in parallel.

The electrode of the present invention may be used as either an anode or a cathode to oxidise or reduce reactants respectively.

Porous diamond compacts have particular application as electrodes in biosensors and especially in applications known as bio-recognition systems such as those for enzymes or antibodies. In such applications, the porous surface of the electrode enables the bio-recognition entity to be immobilised at or near the surface of the electrode by absorption. In prior art applications, the bio-recognition entity is immobilised on the surface of the electrode by the use of an adhesive medium or by enclosing the bio-recognition entity in a membrane. These practices increase the response time and decrease the reproducibility of the electrode.

A further advantage of the porous diamond electrode is that it presents a higher surface area, and hence a greater area for electrochemical activity, than the non-porous equivalent. Thus porous diamond electrodes are suitable for other applications in which these attributes are advantageous. Such applications include, but are not limited to, fuel cell electrodes, wastewater purification systems and the generation of ozone.

The application of the invention is illustrated by the following examples.

### Example 1

A quantity of boron-doped diamond particles containing about 1000ppm boron and having a particle size range of 75 to 90 microns was placed in a tantalum canister, which in turn was placed in an assemble suitable for introduction into a high pressure, high temperature apparatus. The canister was raised to conditions of about 1150°C and 5 GPa, using a temperature-time profile of the form shown in Figure 1. The conditions of maximum temperature and pressure were maintained for a period of 30 minutes. After reducing the conditions to ambient, the diamond compact was recovered by removing the canister material mechanically. The diamond compact, in the form of an irregular disc, was ground to provide two major flat surfaces which were parallel to one another. A regular disc, 5mm diameter, was cut from the piece with the parallel surfaces using a laser. The resulting disc had good mechanical strength. The porosity of the disc was measured to be 14% by volume, and the resistance between the two parallel faces was measured at about 1 ohm.

### Example 2

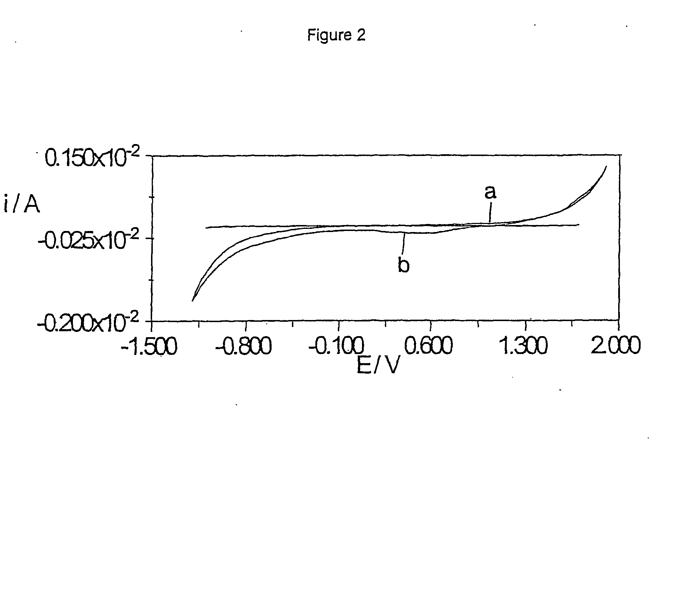

The electrode of Example 1 was mounted into a holder and graphite paste used to make an electrical contact to the external circuitry. The electrode assembly so formed was made an electrode of a standard voltammetry circuit. A cyclic voltammogram was generated for a 0.5 mol/L perchloric acid solution. A conventional CVD diamond electrode of the same dimensions was substituted for the porous diamond electrode of this invention and a cyclic voltammogram generated using another portion of the same perchloric acid solution. Figure 2 shows the two cyclic voltammograms, where curve a is for the electrode of the present invention and curve b is for a standard CVD diamond electrode. The baseline current of the porous diamond electrode is marginally larger than that of the conventional diamond electrode. This is probably due to the larger surface area of the porous diamond electrode due to its porosity. Otherwise, the voltammograms are similar indicating that the porous diamond electrode can be used without significant reduction of sensitivity.

### Example 3

In this Example the porous diamond electrode is used in a "lock and key" type bio-sensory application. In this type of application, the sensing electrode is coated with a bio-recognition entity. Such a system is exemplified by the system for detecting and measuring thyroid hormones (see Analytical Letters vol 33, number 3, pages 447 to 455, 1999).

Another electrode made according to Example 1 was mounted according to Example 2 in the same voltammetry circuit. The porous electrode was pretreated by soaking in a solution of mouse monoclonal anti-T₃ (anti 3,3',5 triiodo L thyronine) or anti-T₄ (anti 3,3',5,5' tetraiodo L thyronine) for the detection of L-T₃ (3,3',5 triiodo L thyronine) and L-T₄ (3,3',5,5' tetraiodo L thyronine) respectively. Using the electrode in chronoamperometric mode, the response times for detection of the thyroid hormones, L-T₃ and L-T₄ were measured. Both response times were found to be approximately 6 seconds. The prior art method of immobilising the monoclonal mouse anti-T₃ and anti-T₄ on a conventional electrode gives response times of 10 to 100 seconds.

## Claims

1. A diamond electrode comprising a polycrystalline mass of diamond particles bonded together and having a contact surface that is, at least partly, porous wherein the porous surface of the electrode is created by leaching non-diamond material, at least in part, from the bonded polycrystalline mass of diamond particles, either before or after shaping it into an electrode, alternatively or additionally wherein the porous surface of the electrode is created by subjecting a mass of diamond particles to conditions of elevated temperature and pressure to self-bond the particles together in the absence of a second phase.

2. A diamond electrode according to claim 1, wherein the non-diamond material leached from the bonded polycrystalline mass of diamond particles is a second phase of a metallic material.

3. A diamond electrode according to any one of the preceding claims, wherein the entire diamond electrode is porous.

4. A diamond electrode according to any one of the preceding claims, wherein the mass of diamond particles contains an appropriate level of an element other than carbon to render the diamond particles electrically conducting.

5. A diamond electrode according to claim 4, wherein the element other than carbon is boron.

6. A diamond electrode according to any one of the preceding claims, wherein the diamond particles are made by a high pressure, high temperature (HPHT) method, by a chemical vapour deposition (CVD) method, or by any other method producing diamond particles which are electrically conducting, or the diamond particles are natural diamond particles.

7. A diamond electrode according to any one of the preceding claims, wherein the size of the diamond particles is generally less than about 1 000 microns.

8. A diamond electrode according to claim 7, wherein the size of the diamond particles is less than about 100 microns.

9. A diamond electrode according to claim 8, wherein the size of the diamond particles is less than about 60 microns.

10. A diamond electrode according to any one of claims 7 to 9, wherein the diamond particles are provided by crushing larger diamond particles or crystals to an appropriate size range.

11. A biosensor comprising a porous diamond electrode according to any one of the preceding claims.

12. A biosensor according to claim 11, which is incorporated into a bio-recognition system.

13. A biosensor according to claim 12, wherein the bio-recognition system is used for detecting enzymes or antibodies.

14. A process for producing a diamond electrode comprising bonding together a polycrystalline mass of diamond particles.

15. A process according to claim 14 comprising the step of producing the diamond particles by crushing larger diamond particles.

## Patentansprüche

1. Diamantelektrode mit einer polylaistallinen Masse aus Diamantpartikeln, die zusammen verbunden sind und eine Kontaktoberfläche aufweisen, die zumindest teilweise porös ist, wobei die poröse Oberfläche der Elektrode erzeugt wird, indem nicht aus Diamant bestehendes Material zumindest teilweise aus der verbundenen polykristallinen Masse aus Diamantpartikeln entweder vor oder nach ihrer Formung zu einer Elektrode herausgelangt wird, wobei, alternativ oder zusätzlich, die poröse Oberfläche der Elektrode erzeugt wird, indem eine Masse aus Diamantpartikeln Bedingungen hoher Temperatur und hohen Drucks ausgesetzt wird, um die Partikel in Abwesenheit einer zweiten Phase untereinander zu verbinden.

2. Diamantelektrode nach Anspruch 1, wobei das nicht aus Diamant bestehende Material, welches aus der verbundenen polykristallinen Masse aus Diamantpartikeln herausgelaugt wird, eine zweite Phase aus einem metallischen Material ist.

3. Diamantelektrode nach einem der vorangehenden Ansprüche, wobei die gesamte Diamantelektrode porös ist.

4. Diamantelektrode nach einem der vorangehenden Ansprüche, wobei die Masse aus Diamantpartikeln eine geeignete Konzentration eines Elements enthält, das sich von Kohlenstoff unterscheidet, um die Diamantpartikel elektrisch leitend vorzusehen.

5. Diamantelektrode nach Anspruch 4, wobei das Element, das sich von Kohlenstoff unterscheidet, Bor ist.

6. Diamantelektrode nach einem der vorangehenden Ansprüche, wobei die Diamantpartikel durch ein Hochdruck-Hochtemperaturverfahren (HPHT-Verfahren), durch ein chemisches Dampfabscheidungsverfahren (CVD-Verfahren) oder mittels eines beliebigen weiteren Verfahrens hergestellt wird, das Diamantpartikel erzeugt, die elektrisch leitend sind, oder wobei die natürliche Diamantpartikel sind.

7. Diamantelektrode nach einem der vorangehenden Ansprüche, wobei die Größe der Diamantpartikel im Allgemeinen kleiner als ungefähr 1000 Mikron ist.

8. Diamantelektrode nach Anspruch 7, wobei die Größe der Diamantpartikel kleiner als ungefähr 100 Mikron ist.

9. Diamantelektrode nach Anspruch 8, wobei die Größe der Diamantpartikel kleiner als ungefähr 60 Mikron ist.

10. Diamantelektrode nach einem der Ansprüche 7 bis 9, wobei die Diamantpartikel vorgesehen werden, indem größere Diamantpartikel oder Kristalle auf einen geeigneten Größenbereich zerkleinert werden.

11. Biosensor mit einer porösen Diamantelektrode nach einem der vorangehenden Ansprüche.

12. Biosensor nach Anspruch 11, der in einem Bio-Erkennungssystem enthalten ist.

13. Biosensor nach Anspruch 12, wobei das Bio-Erkennungssystem verwendet wird, um Enzyme oder Antikörper zu detektieren.

14. Prozess zum Herstellen einer Diamantelektrode, umfassend: Miteinanderverbinden einer polykristallinen Masse aus Diamantpartikeln.

15. Verfahren nach Anspruch 14, welches den Schritt des Erzeugens der Diamantpartikel durch Zerkleinern größerer Diamantpartikel umfasst.

## Revendications

1. Electrode de diamant comprenant une masse polycristalline de particules de diamant liées ensemble et ayant une surface de contact qui est, au moins partiellement, poreuse dans laquelle la surface poreuse de l'électrode est créée par lixiviation d'un matériau exempt de diamant, au moins en partie, à partir de la masse polycristalline de particules de diamant liées, soit avant soit après façonnage de celle-ci en une électrode, dans laquelle en variante ou en plus la surface poreuse de l'électrode est créée par soumission d'une masse de particules de diamant à des conditions de température et de pression élevées pour lier ensemble automatiquement les particules en l'absence d'une seconde phase.

2. Electrode de diamant selon la revendication 1, dans laquelle le matériau exempt de diamant lixivié à partir de la masse polycristalline de particules de diamant liées est une seconde phase d'un matériau métallique.

3. Electrode de diamant selon l'une quelconque des revendications précédentes, dans laquelle la totalité de l'électrode de diamant est poreuse.

4. Electrode de diamant selon l'une quelconque des revendications précédentes, dans laquelle la masse de particules de diamant contient un niveau approprié d'un élément autre que du carbone pour rendre les particules de diamant électriquement conductrices.

5. Electrode de diamant selon la revendication 4, dans laquelle l'élément autre que le carbone est du bore.

6. Electrode de diamant selon l'une quelconque des revendications précédentes, dans laquelle les particules de diamant sont fabriquées au moyen d'un procédé haute pression, haute température (HPHT), au moyen d'un procédé de dépôt chimique en phase vapeur (CVD), ou au moyen d'un autre procédé quelconque produisant des particules de diamant qui sont électriquement conductrices, ou les particules de diamant sont des particules de diamant naturelles.

7. Electrode de diamant selon l'une quelconque des revendications précédentes, dans laquelle la granulométrie des particules de diamant est généralement inférieure à environ 1 000 micromètres.

8. Electrode de diamant selon la revendication 7, dans laquelle la granulométrie des particules de diamant est inférieure à environ 100 micromètres.

9. Electrode de diamant selon la revendication 8, dans laquelle la granulométrie des particules de diamant est inférieure à environ 60 micromètres.

10. Electrode de diamant selon l'une quelconque des revendications 7 à 9, dans laquelle les particules de diamant sont mises à disposition par broyage de particules de diamant ou de cristaux de plus grande taille jusqu'à une plage de granulométrie appropriée.

11. Biocapteur comprenant une électrode de diamant poreuse selon l'une quelconque des revendications précédentes.

12. Biocapteur selon la revendication 11, qui est incorporé dans un système de reconnaissance biologique.

13. Biocapteur selon la revendication 12, dans lequel le système de reconnaissance biologique est utilisé pour détecter des enzymes ou des anticorps.

14. Processus de fabrication d'une électrode de diamant comprenant l'étape consistant à lier ensemble une masse polycristalline de particules de diamant.

15. Processus selon la revendication 14, comprenant l'étape consistant à produire les particules de diamant par broyage de particules de diamant de plus grande taille.
